# EUROPEAN PATENT SPECIFICATION

(11) **EP 4 619 083 B1**
(45) Date of publication and mention of the grant of the patent: **25.03.2026**
(21) Application number: 22844261.2
(22) Date of filing: 16.11.2022
(51) Int. Cl.: A61N 1/05, A61B 17/28

(54) **MULTI-PIN FIXATION TOOL**
BEFESTIGUNGSWERKZEUG MIT MEHREREN STIFTEN
OUTIL DE FIXATION À BROCHES MULTIPLES

(43) Date of publication of application: 24.09.2025
(73) Proprietor: Sorin CRM SAS, 92140 Clamart (FR)
(72) Inventor: VIDAL, Clément, 92140 Clamart (FR); SHAN, Nicolas, 92140 Clamart (FR); OLLIVIER, Jean-François, 92140 Clamart (FR); DAVID, Benoît, 92140 Clamart (FR)
(74) Representative: Gulde & Partner
(86) International application number: PCT/IB2022/000677
(87) International publication number: WO 2024/105424

(56) References cited:
- US-A1- 2012 022 356
- US-A1- 2012 130 397
- US-B1- 8 147 275
- US-B2- 6 842 649
- US-B2- 7 753 696

## Description

### Field

The present application relates to lead fixation tool - such as but not limited to - a medical clip for clamping and rotating a lead terminal pin of a medical implantable cardiac lead.

### Background

Implantable medical devices, such as cardiac rhythm management (CRM) devices and neuromodulation devices, are used in a variety of therapeutic applications. In some applications, one or more medical implantable cardiac leads ("leads" in the following) are employed to deliver therapy from an implanted medical device to tissues within a body. CRM systems may employ electrical leads implanted within a patient's heart. Such leads may be secured to a desired location in the heart by a mechanical fixation device. Such mechanical fixation devices may include a corkscrew-shaped device known as a helix (which also may be an electrode of the lead). The helix may be designed such that it is retracted into the lead during insertion and positioning within the heart. Once positioned, the helix is rotated to extend the helix and screw it into the heart muscle.

Helix rotation at the distal end of the lead may be driven by torque applied to a lead terminal pin located at the proximal end of the lead and transmitted through a conductor coil extending through the lead from the terminal pin to the helix. Lead terminal pins are often quite small and have smooth, cylindrical surfaces. This presents some difficulty in applying a desired amount of torque to ensure a number of rotations necessary to properly seat the helix.

The lead terminal pin may be mechanically and electrically connected to a distal retractable screw (e.g., helical electrode) of a lead. By operating the lead terminal pin, the distal retractable screw can be retracted or extended from a distal end of the lead. It allows, in particular, anchoring the distal retractable screw into cardiac tissue so as to secure the distal end of the lead. Hence, a rotation of the lead terminal pin can allow screwing the distal retractable screw into cardiac tissue.

For rotating the lead terminal pin, a fixation tool (e.g., medical clip) may be used to allow a clinician to clamp and transmit torque to the lead terminal pin. This presents some difficulty in applying a desired amount of torque to ensure a number of rotations necessary to properly seat the helix, especially in case different lead terminal pins are provided for different types of leads. Documents US 6,842,649 and US 2012/022356 both disclose known devices.

### Summary

The present invention addresses the above-mentioned object by providing a fixation for clamping and rotating a lead terminal pin of a medical implantable cardiac lead according to claim 1.

According to an aspect of the present invention, the proposed fixation tool comprises:
a handle portion comprising a pair of arms formed by a first arm connected at a connecting hinge portion to a second arm, and
a jaw portion comprising a pair of jaws for receiving the terminal pin between respective inner surfaces of the pair of jaws,
the connecting hinge portion being arranged between the jaw portion and the handle portion,
wherein a pressure exerted on the first and second arms causes an opening of the pair of jaws by means of a pivotal movement of the first arm and the second arm about the connecting hinge portion; and
wherein opposing inner surfaces of the pair of jaws are shaped to form a cavity system between the pair of jaws, the cavity system comprising:
   a first cavity located proximal to an opening at the distal end of the jaw portion and having a first width between the opposing inner surfaces and being shaped to clamp a first lead terminal pin of a first diameter;
   a second cavity located proximal to the first cavity and having a second width between the opposing inner surfaces, smaller than the first width and adapted to clamp, together with the first cavity, a second lead terminal pin of a second diameter larger than the first diameter; and
   a third cavity located proximal to the second cavity and having a third width smaller than the first diameter.

Thus, the proposed cavity system with communicating first and second cavities allows to clamp and centre lead terminal pins of various diameters and thus facilitates use of the fixation tool for various different types of lead terminal pins (e.g., IS1, IS4, DS1, DS4, etc.). Lead terminal pins with larger diameters can be centred by contact points (e.g., edges) obtained from the combined enlarged cavity of the communicating first and second cavities.

According to a first option that can be combined with the above aspect, the cavity system may comprise a fourth cavity located proximal to the third cavity and forming a proximal end of the cavity system and having a width larger than the third width. Thereby, the proximal end of the cavity system can be used as a stress relieving element for the jaw portion.

According to a second option that can be combined with the first option or the above aspect, each of the first and second arms may comprise a finger pad having a concave, a convex or a flat surface at an external surface of the handle portion. A specific region of the first and second arms is thereby precisely designed for receiving a pressure applied by means of fingers. This clearly and intuitively indicates to a clinician the more adapted and ergonomic region of the fixation tool for pressing the medical clip in order to open the jaw portion. Thus, by means of the finger pads specifically dedicated for receiving a finger, the ergonomic can be enhanced.

Moreover, a parallel arrangement of the finger pads allows facilitating further the handle of the medical clip, in particular for pressing the arms at the handle portion. It is indeed easier for a clinician to press parallel surfaces than inclined surfaces with respect to each other, if only for preventing the possibility of fingers slipping, improving handling characteristics. The parallel arrangement of the finger pads also allows improving the pressure distribution applied by the clinician. Hence, the presence of finger pads and their parallel arrangement provide a more intuitive medical clip to a clinician.

In a specific example of the second option, a surface of each finger pad may be provided with an anti-slip surface, in particular with a ribbed surface and/or a rubber layer. Thereby, reliable and proposer use of the handle portion can be supported e.g. by preventing slipping of the fingers during use.

According to a third option that can be combined with the first or second option or the above aspect, each of the first and second arms may be curved and the finger pad of each of the first and second arms may be positioned at an inflection point of the respective arm at the handle portion. Thus, the resultant enlarged gap between the two arms allows for wider opening and round shape for allowing rotational movement with the finger(s) of the user.

According to a fourth option that can be combined with any one of the first to third options or the above aspect, the internal surface of each of the first and second arms at the handle portion opposite to the finger pad is provided with a concave portion adapted to receive a finger for rotating the fixation tool with respect to the terminal pin. Thereby, the design of the arms provides a space with a round shape between the arms, that facilitates rotational movement of the fixation tool by a finger for a screwing operation.

According to a fifth option that can be combined with any one of the first to fourth options or the above aspect, the internal surface of the first arm may be provided with a first protrusion and the internal surface of the second arm may be provided with a second protrusion, the first protrusion and the second protrusion being arranged such that a predetermined pressure on the handle portion causes said protrusions to abut against each other to provide a stopping function. Thereby, the amount of rotational movement (opening/closing) of the jaws of the jaw portion can be limited to prevent excessive material stress e.g. at the connecting hinge portion.

According to a sixth option that can be combined with any one of the first to fifth options or the above aspect, the first protrusion and the second protrusion may be arranged at a free end of the handle portion. Thus, the end portions of the arms of the handle portion can be used to provide the stopping function.

According to a seventh option that can be combined with any one of the first to fifth options or the above aspect, the first protrusion and the second protrusion may be arranged between the finger pads and the connecting hinge portion. Thus, the end portions of the arms can be shortened to provide a larger opening at the proximal end of the handle portion, which facilitates access to the inner space between the arms for rotating the fixation tool.

According to an eighth option that can be combined with any one of the first to seventh options or the above aspect, the opening of the distal end of the jaw portion may be smaller than the second diameter when no pressure is applied to the handle portion. Thereby, an insertion force is required to insert the lead terminal pin into the opening of the jaw portion and to provide a click function during insertion.

According to a ninth option that can be combined with any one of the first to eighth options or the above aspect, the first diameter may range between 0.50mm and 1.20mm, in particular between 1.62mm and 1.56mm, and the second diameter may range between 0.50mm and 1.30mm, in particular between 1.40mm and 1.34mm, when no pressure is applied to the handle portion. Thereby, the fixation tool can be adapted for combined use of e.g. IS1 and DF4 lead terminal pins or other terminal pins of similar diameters.

According to a tenth option that can be combined with any one of the first to ninth options or the above aspect, the minimum distance between the opposing inner surfaces of the pair of jaws at the opening of the distal end may range between 0.50mm and 1.20mm, in particular between 0.80mm and 1.00mm, when no pressure is applied to the handle portion. Thereby, a desired click function can be provided for a combined use of e.g. IS1 and DF4 lead terminal pins or other terminal pins of similar diameters.

According to an eleventh option that can be combined with any one of the first to tenth options or the above aspect, the fixation tool may be integrally made in one-piece, in particular of a plastic material. Thereby, manufacturing costs can be reduced.

According to a twelfth option that can be combined with any one of the first to eleventh options or the above aspect, the fixation tool may further comprise an insertion support funnel removably or non-removably fixed to the jaw portion to facilitate insertion of a stylet into the cardiac lead via an aperture of the lead terminal pin.

### Brief Description of the Drawings

To assist understanding of the present disclosure and to show how embodiments may be put into effect, reference is made by way of example to the accompanying drawings in which:
Fig. 1A schematically shows how a fixation tool is clamped to terminal pin of a lead;
Fig. 1B schematically shows how the fixation tool of Fig. 1A is rotated to screw the distal helix into the heart tissue;
Fig. 2 schematically shows a side view of a fixation tool with finger pads, according to an embodiment;
Figs. 3A and 3B schematically show perspective views of two alternative examples of the handle portion of the fixation tool according to embodiments;
Fig. 4 schematically shows a perspective view of fixation tool with alternative stopping protrusions according to an embodiment;
Fig. 5 schematically shows an enlarged portion of the fixation tool according to an embodiment with a cavity system comprising four cavities;
Fig. 6 schematically shows enlarged jaw portions of a fixation tool according to an embodiment with different lengths of the third cavity;
Fig. 7 schematically shows an enlarged jaw portion of a fixation tool according to an embodiment with inserted lead terminal pins of different diameter;
Fig. 8 schematically shows an enlarged jaw portion of a fixation tool according to an embodiment with an indication of a width of an opening at a distal end;
Fig. 9 schematically shows a perspective view of a cavity system at a jaw portion of a fixation tool according to an embodiment with inclined opposing inner jaw surfaces;
Fig. 10 schematically shows a perspective view and a front view of a fixation tool with an insertion support funnel according to an embodiment; and
Fig. 11 schematically shows a perspective view and a front view of a fixation tool with an alternative design of the insertion support funnel.

### Detailed Description

In the following, embodiments of the present invention are described in more detail based on a fixation tool (e.g., medical clip) which enables a physician or other user to screw a distal electrode (e.g., helix) of a lead into the tissue of a patient's heart. More specifically, the physician is able to clamp/remove the fixation tool to/from a terminal pin (e.g., an IS1 and/or a DF4 pin) of the lead and to transmit torque to the terminal pin in an intuitive manner.

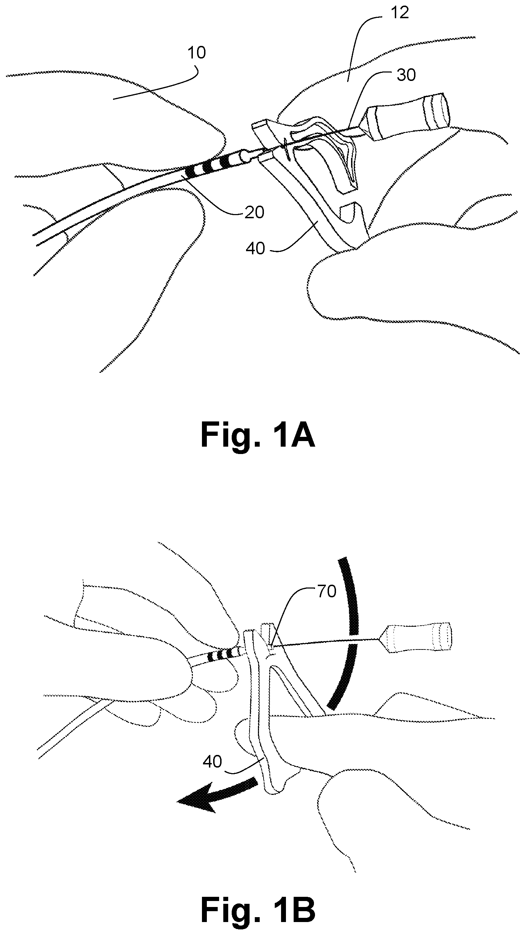

Fig. 1A schematically shows how a butterfly-shaped fixation tool 40 is clamped to lead terminal pin 70 (shown in Fig. 1B only) provided at an electrical conductor of a lead 20.

In order to ensure a safe pin insertion, wings (arms) of a proximal handle portion of the fixation tool 40 are required to be pressed by the fingers 12 of one hand of a user to open a cavity system provided between a pair of jaws of a distal jaw portion, while the lead 20 may be held e.g. by fingers 10 of the other hand for pin-cavity insertion so as to clip the fixation tool 40 to the terminal pin.

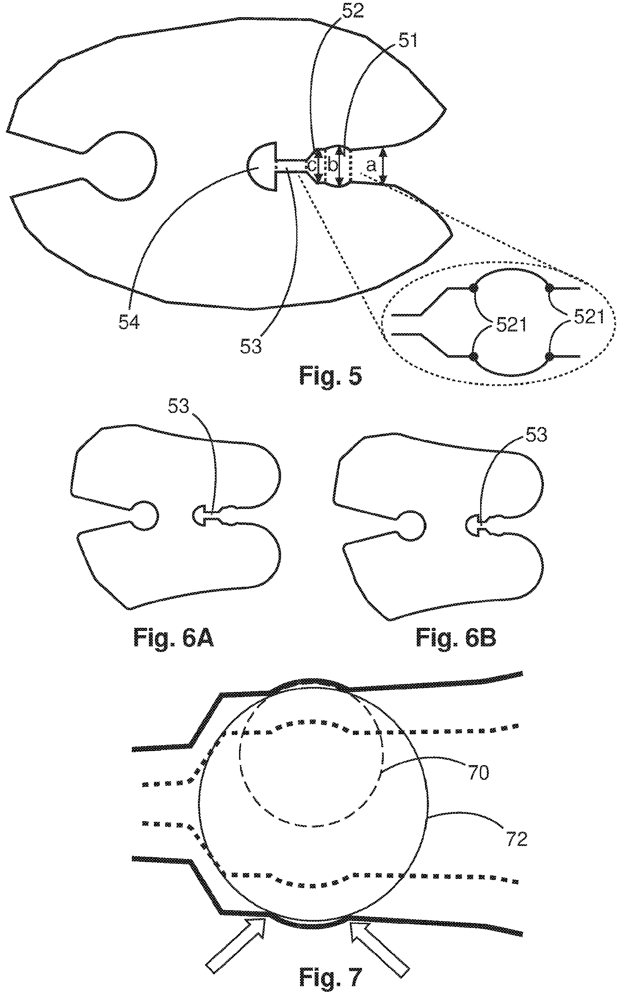

An example of the cavity system is described later in more detail cavity with reference to Fig. 5.

Fig. 1B schematically shows how the fixation tool 40, after being clipped to the lead terminal pin 70, is rotated with a finger of the user with an appropriate number of turns to screw a distal helix (not shown) of the lead 20 into heart tissue.

Finally, the fixation tool 40 is removed from the terminal pin by pressing the wings of the handle portion again.

The proximal end of the lead 20 may include a terminal boot and the terminal pin. The terminal boot may be made of an elastic polymer and may include one or more larger diameter terminal boot seals for sealing the terminal boot to an implantable electrical device (not shown). The lead terminal pin 70 may be a metallic cylinder having a relatively smooth surface for electrically connecting the conductor of the lead 20 to the implanted medical device. The electrical conductor may be disposed within a lumen within the body of the lead 20 and extend from the lead terminal pin 70 to the helix to provide both a physical connection and, in some embodiments, an electrical connection between the lead terminal pin 70 and the helix electrode. The conductor may be at least partly in the form of a coil within the lead 20.

The lead terminal pin 70 may rotate freely relative to the terminal boot and the body of the lead 20 to transmit torque to the helix via the conductor. In the embodiment of Figs. 1A and 1B, the lead terminal pin 70 also includes an aperture extending axially through the lead terminal pin 70. The conductor also defines a lumen in communication with the aperture such that a stylet 30 can be inserted from the proximal end towards the distal end of the lead 20 to assist in positioning the distal end and the helix within the heart.

In some embodiments, the body of the lead 20 may be a tubular structure including one or more lumens (not shown). The distal end may include at least one electrode (not shown) and the helix electrode. The electrodes may be employed to electrically couple the lead 20 with a patient's heart (not shown). The helix 20 may also be used to electrically couple the lead 20 with the heart.

As already mentioned above, torque can be applied to the lead terminal pin 70 by using the fixation tool 40 to rotate the helix and anchor the distal end of the lead 20 in the heart. For each type of the lead 20, a specific number of rotations of the lead terminal pin 70, and a corresponding number of rotations of the helix, are necessary to successfully anchor the distal end in the heart tissue. Thus, the fixation tool 40 is employed to grip the lead terminal pin 70 and rotate it, while the number of rotations is counted to determine when the helix is successfully implanted.

Pacemaker or defibrillator leads connect the myocardial site of sensing or stimulation with the respective cardiac implantable electronic devices (CIEDs) or other medical devices. When replacing a CIED, the leads may remain in place and may be mounted individually to a new medical device. Occasionally, additional leads may be placed to address sensing or capture issues with existing leads. Since the late 1980s and early 1990s, unified industry standards IS1 and DF1 ensure interchangeability of generators and leads from different manufacturers, thus allowing the device system to be tailored to the individual patient's needs. Such leads may thus possess a bifurcation (single-coil lead; one IS1, one DF1) or a trifurcation (dual-coil leads; one IS1, two DF1) at their proximal end.

A new standard was formalized in March 2010, when the International Organization for Standardization (ISO) published its new standard ISO 27186.1 for active implantable medical device with four-pole connector system for implantable cardiac rhythm management devices. Its specifications apply to both low-energy (IS4) and high-energy (DF4) leads and ensures that IS4/DF4 leads are compatible with future implanted devices.

Therefore, different lead terminal pins (e.g., IS1 and DF4 pins) have different designs with a resulting risk of mistakes (e.g., lead pin break at insertion) by using a wrong fixation tool.

Various embodiments of an improved fixation tool 40 with modified design for easier pin insertion when the fixation tool is open, more intuitive use and compatibility for different lead pin types (e.g., IS1, DF4) are now described.

The opening of the jaw portion of the fixation tool 40 is configured to be wide enough for free pin insertion with less insertion force and a click function.

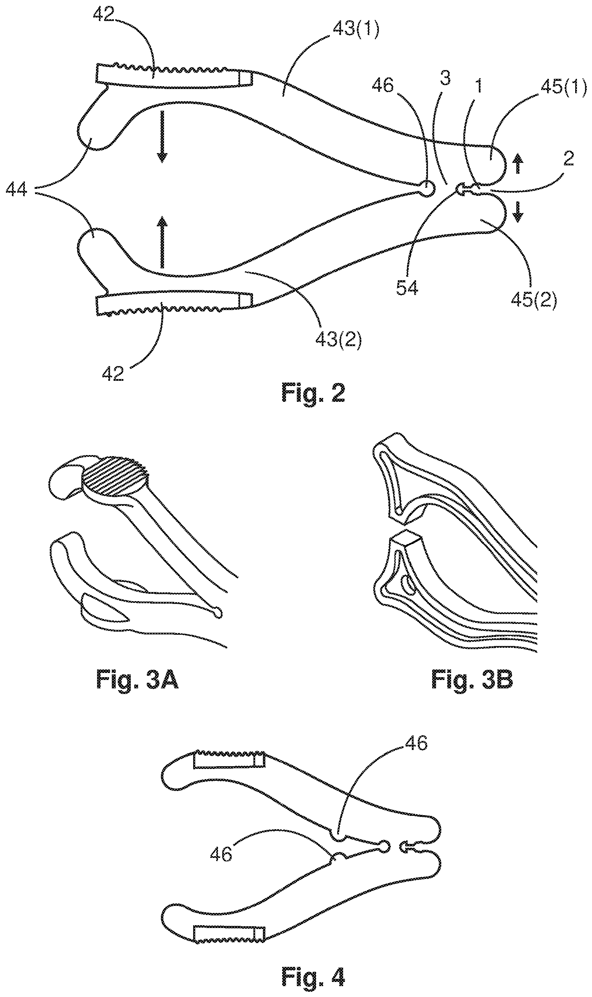

Fig. 2 schematically shows a side view of a fixation tool with finger pads 42, according to an embodiment.

The fixation tool includes a handle portion with a first arm 43(1), a second arm 43(2), a connecting hinge portion 3, a jaw portion with a pair of jaws including a first jaw 45(1), a second jaw 45(2), a jaw opening 2 and a cavity system 1 configured to clamp at least two different types of lead terminal pins. Protruding end portion portions 44 of the first and second arms 43(1) and 43(2) serve to provide a stopping function (hard stop) when the first and second arms 43(1) and 43(2) are pressed together (as shown by the larger arrows in Fig. 2) e.g. by the fingers of a user. Under pressure, the connecting hinge portion 3 serves as an elastic hinge that rotates the first and second jaws 45(1) and 45(2) in opposite directions to increase the opening 2 of the jaw portions (as shown by the smaller arrows in Fig. 2).

The first arm 43(1), the second arm 43(2), the connecting hinge portion 3, the first jaw 45(1), and the second jaw 45(2) may be integrally formed of a first material. In other embodiments, the connecting hinge portion 2 may be formed of a different material having greater elasticity than the first material. The first jaw 45(1) may project beyond the connecting hinge portion 3 from the first arm 43(1). The second jaw 45(2) may project beyond the connecting hinge portion 3 from the second arm 43(2).

As shown in Fig. 2, the first jaw 45(1) and the second jaw 45(2) are spaced apart, forming a tool slot with the opening 2 and the cavity system 1. The tool slot extends from end portions of each of the first jaw 45(1) and the second jaw 45(2) to a distal end of the connecting hinge portion 3. The connecting hinge portion 3 may be a portion of the first material connecting the first arm 43(1) to the second arm 43(2) and may include a handle stress relieving element 46 and a jaw stress relieving element 54. The handle stress relieving element 46 may be a curved cut-out feature (e.g., a cavity with circular or other shape) formed where the first arm 43(1) and the second arm 43(2) connect to the connecting hinge portion 3 to reduce a concentration of stress in the connecting hinge portion 3. The jaw stress relieving element 54 may also be a curved cut-out feature (e.g., a cavity with half-circular or other shape) formed as a proximal part of the cavity system 1 where the first jaw 45(1) projects from the first arm 43(1) and the second jaw 45(2) projects from the second arm 43(2) to reduce a concentration of stress in the connecting hinge portion 3.

The cavity system 1 may be disposed at or near the opening 2 of the jaw portion, may extend to the connecting hinge portion 3, and may include the jaw stress relieving element 54 as one of its cavities. The opening 2 of the jaw portion is configured to align with the cavity system 1 which is shaped for clamping at least two kinds of lead terminal pins with different diameters by using at least two of a plurality of communicating cavities formed by opposing inner surfaces of the first and second jaws 45(1), 45(2).

The cavity system 1 with the opening 2 may be disposed in the fixation tool by insert molding. That is, the pattern of cavity system 1 with the opening 2 may be placed in a mold and the rest of fixation tool may be injection molded around the cavity system 1 with the opening 2. Alternatively, the cavity system 1 with the opening 2 may be glued to or stamped out of the first jaw 45(1) and the second jaw 45(2).

The tool slot with the cavity system 1 and the opening 2 may be configured such that pressing the first arm 43(1) toward the second arm 43(2) causes a movement of the first jaw 45(1) and the second jaw 45(2) relative to each other to change a width of the cavity system 1. In the embodiment shown in Fig. 2, the tool slot is configured such that pressing the first arm 43(1) toward the second arm 43(2) causes the first jaw 45(1) and the second jaw 45(2) to move farther from each other and against a restoring force of the connecting hinge portion 3. In doing so, the width of the tool slot and thus the width of the cavity system 1 and the opening 2 increase. In some embodiments, the width of the cavity system 1 may increase from less than a smallest diameter of at least two different clampable terminal pins to a width greater than the largest diameter of the at least two different clampable terminal pins.

As already mentioned above, the first jaw 45(1) and the second jaw 45(2) may be made of the first material. The surface area of the insert slot with the cavity system 1 and the opening 2 may be made of a second material. The first material may be harder than the second material. For example, the first material may be a thermoplastic of relative high durometer, such as a polycarbonate or acrylonitrile butadiene styrene (ABS) having a Shore hardness of greater than 90D. The second material may be a thermoplastic, thermoset, or cast material with a Shore hardness less than 90D. The second material may be, for example, silicone rubber with a Shore hardness less than 90D.

Figs. 3A and 3B schematically show perspective views of two alternative examples of the handle portion of the fixation tool according to embodiments for more intuitive use and simplified shape.

Fig. 3A shows the example of Fig. 2 with the finger pads at an inflection point of the first and second arms, while Fig. 3B shows a modification of the tool arms without finger pads and scissor-like arms. In both cases, the enlarged gap between the two arms allows for wider opening and round shape for allowing rotational movement with the finger(s) of the user.

Fig. 4 schematically shows a perspective view of fixation tool with alternative location of stopping protrusions 46 according to an embodiment. Placing the stopping protrusion towards the connecting hinge portion allows for shorter arms (which now look more like pliers), so that insertion of finger(s) from the back side for rotational movement is facilitated.

Fig. 5 schematically shows an enlarged cut-out portion of an example of the cavity system of the fixation tool according to an embodiment, wherein the proximal end of the cavity system on the horizontal axis of Fig. 5 is located at connecting hinge portion and the distal end of the cavity system is located at the opening of the jaw portion. The cavity system is formed by the opposing inner surfaces of the first and second jaws of the jaw portion and comprises four successive communicating cavities 51 to 54 (indicated by dotted separation lines in Fig. 5) configured to clamp two or more different types of lead terminal pins with different diameters.

A first cavity 51 is located proximal to the opening of the jaw portion and has a first width *b* between the opposing inner surfaces, adapted to clamp a first type of lead terminal pin with a first diameter. Additionally, a second cavity 52 is located proximal to the first cavity 51 and has a second width *c* between the opposing inner surfaces, smaller than the first width *b* and adapted to clamp together with the first cavity 51 a second type of lead terminal pin with a second diameter larger than the first diameter. Thus, the combined shape of the neighboring communicating first and second cavities 51, 52 can be used to clamp a larger lead terminal pin, wherein four edge portions 521 (shown in a zoomed part of Fig. 5) created by the distal shape of the first cavity 51 and the combination of the different first and second cavities 51, 52 at the proximal end of the first cavity 51 serve to autocentre the larger second type of terminal lead together with the contact points created at the distal end of the first cavity 51. Thus, in the example of Fig. 5, the larger lead terminal pin is centred by the four contact points 521.

Moreover, an additional third cavity 53 is located proximal to the second cavity 52 and has a third width smaller than the second diameter to prevent that the smaller first type of lead terminal is inserted beyond the second cavity 53.

Finally, a fourth cavity 54 (which may correspond to the jaw stress relieving element of Fig. 2) may be located proximal to the third cavity 53, forms a proximal end of the cavity system (1) and has a width larger than the third width. The fourth cavity 54 facilitates opening and closing of the jaw portion during a clamping action. Various other shapes (e.g., full circular shape) of the fourth cavity 54 can be implemented, although a non-circular shape (e.g., D-shape or halfmoon shape as shown in Fig. 5) prevents a physician or other user from accidentally inserting a lead terminal pin into the fourth cavity 54.

The opening of the jaw portion may have a third width *a* which may be smaller than the first and second widths *b* and *c* of the first and second cavities 51, 52 to ensure that none of the two lead terminal pins can be accidentally inserted in the non-compressed state of the arms of the fixation tool. The second width *c* may be equal to the third width *a* to facilitate manufacturing of the structures at the opposing inner surfaces of the first and second jaws.

Accordingly, the cavity system is formed by a plurality of structures projecting from the inner surfaces of the first and second jaws to define the above cavities 51 to 54 and thereby allow securing the fixation tool to a respective one of two or more types of terminal pins with different diameters by providing adapted surface contact areas and contact points between a selected terminal pin and the fixation tool. Additionally or alternatively, the inner surfaces may be structured to provide a click function during insertion of a selected lead terminal pin by pushing the lead terminal pin from the second cavity 52 towards the first cavity 51 via the contact points 521.

In an example of a fixation tool adapted for use in connection with IS1 and DF4 lead terminal pins or other terminal pins with similar diameters, the width (distance) of the opening of the jaw portion when no pressure is applied to the handle portion may be selected to range between 1.20mm and 0.50mm, in particular between 1.00mm and 0.80mm. Here, the diameter of the larger terminal pin (e.g., IS1 pin) ranges between 1.50mm and 1.65mm, in particular between 1.56mm and 1.62mm, while the diameter of the smaller terminal pin (e.g., DF4 pin) ranges between 1.30mm and 1.50mm, in particular between 1.34 and 1.40mm. The largest width *b* of the first cavity 51 may then be selected to range between 1.30mm and 1.00mm, in particular between 1.25mm and 1.05mm, to achieve desired clamping and insertion forces.

In Fig. 5, the first cavity 51 is illustrated as having a circular shape. However, it is understood that in other embodiments, the first cavity 51 or the other cavities 52 to 54 or further cavities for further lead terminal pins may have a shape that is a square, a triangle, or other polygon.

Fig. 6 schematically shows enlarged jaw portions of a fixation tool according to an embodiment with different lengths of the third cavity 53 in the pin insertion direction for controlling pressing and clamping behaviour of the fixation tool. In the above example of the IS1/DF4 fixation tool, these different lengths of the third cavity 53 may range e.g. from 1mm to 1.5mm or from 1.5mm to 2mm depending on the desired opening and clamping force characteristics of the jaw portion.

Fig. 7 schematically shows an enlarged jaw portion of a fixation tool according to an embodiment with indicated locations of different inserted lead terminal pins 70, 72 of different diameter. The dotted shape of the cavity system shown in Fig. 7 indicates the location (distance) of the opposing surfaces of the first and second jaws of the jaw portion when no pressure is applied to the handle portion. Note that only one of the two lead terminal pins 70, 72 can be inserted at a time. In the case shown in Fig. 7, the larger lead terminal pin 72 is clamped by the jaw portion, while the smaller lead terminal pin 70 is merely shown for comparison reasons.

The smaller terminal pin 70 can be accommodated in the first cavity alone, wherein the outer shape of the smaller terminal pin 70 is matched to the upper and lower shape of the first cavity created by the opposing inner surfaces of the first and second jaws. By contrast, the larger terminal pin 72 is accommodated in the combined space of the first and second cavity, since the outer shape of the larger terminal pin 72 is not matched to (i.e., larger than) the upper and lower shape of the first cavity. However, the larger terminal pin 72 is automatically centred by four contact points (contact points 521 in Fig. 5) created by the communicating cavities of the cavity system at the proximal and distal ends of the first cavity. The contact points at the surface of the lower jaw in are indicated by respective arrows in Fig. 7.

As already mentioned above, the combined provision of the first and second cavities allows to use the fixation tool for applying torque to two or more different types of terminal pins (e.g., IS1, DF4, etc.) with different diameters.

Referring again to Fig. 2, the first arm 43(1) and the second arm 43(2) may be pressed toward each other, so that the movement of the first arm 43(1) and the second arm 43(2) works against a restoring force of the elastic connecting hinge portion 3 moving the first jaw 45(1) and the second jaw 45(2) apart, increasing the width of the tool slot and the cavity system 1 with opening 2 from less than the smallest diameter of all clampable terminal pins 70, 72 to greater than the largest diameter of all clampable terminal pins 70, 72. Once the tool slot and the cavity system 1 with the opening 2 have a width close to or greater than the diameter of a selected terminal pin that is currently intended to be gripped, the terminal pin may be pressed or slid through the opening 2 and clipped into a respective cavity space of matching width of the cavity system 1.

As shown in Fig. 7, the larger terminal pin 72 can be clamped in a combined cavity space created by the first and second cavities (e.g., the first and second cavities 51 and 52 of Fig. 5) with contact points centring the diameter of the larger terminal pin 72, through a clip function achieved by the smaller diameter of the opening 2. Alternatively, the smaller terminal pin 70 can be inserted and clipped into the first cavity (e.g., the first cavity 51 of Fig. 5) with the smaller width and upper and lower boundary shape adapted to the diameter of the smaller terminal pin 70.

Releasing the first arm 43(1) and the second arm 43(2) allows the restoring force of the connecting hinge member 3 to move the first jaw 45(1) and the second jaw 45(2) closer together, decreasing the width of the respective cavity of the cavity system 1 to approximately the diameter of the selected terminal pin 70 or 72. Because the width of the respective cavity of the cavity system 1 cannot return to the original width of less than the diameter of the selected terminal pin 70 or 72 due to the presence of the selected terminal pin 70 or 72, sufficient restoring force of the connecting hinge portion 3 remains to secure the fixation tool to the selected terminal pin 70 or 72. Once secured to the selected terminal pin, the fixation tool may be employed to apply torque to the selected terminal pin 70 or 72, e.g., as shown in Fig. 1B.

Securing the fixation tool to the selected terminal pin 70 or 72 may be enhanced by a lower hardness of the second material provided at the opposing inner surfaces of the first and second jaws 45(1), 45(2). The second material may grip the terminal pin 70 or 72 more effectively than, for example, the harder first material, because it may deform against the terminal pin 70 or 72 to a greater extent, thereby increasing a surface contact area between the terminal pin 70 or 72 and the fixation tool. The second material may also have a greater coefficient of friction than the first material. These features may provide for a more secure mechanical connection between the fixation tool and the terminal pin 70 or 72.

In an example of a fixation tool adapted for use in connection with IS1 and DS4 terminal pins, a maximum force to open the jaw portion may be selected to about 15N, while a maximum insertion force for inserting the lead terminal pins (click function) via the opening 2 of the jaw portion may be obtained at about 30N. Then, a clamping force of about 30N may be exerted on the inserted terminal pin.

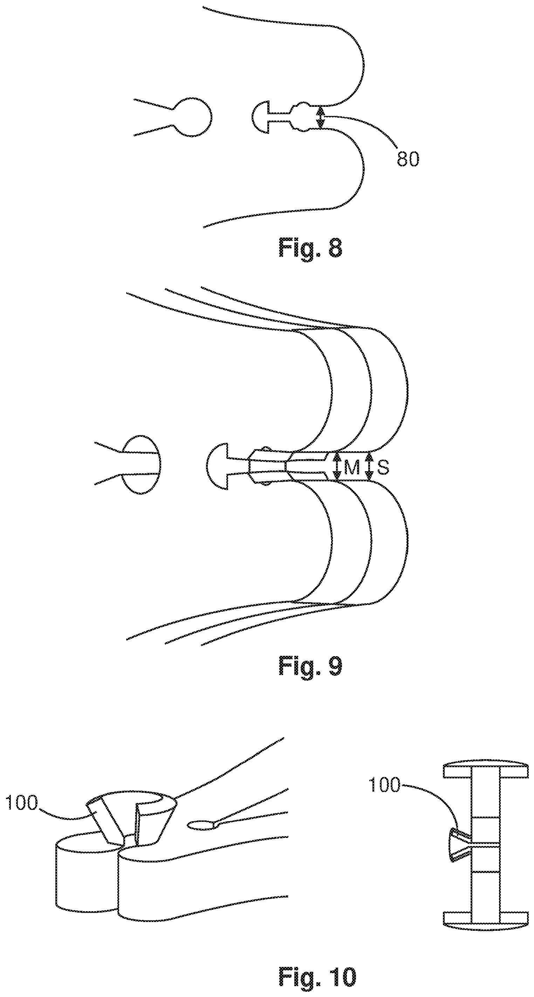

Fig. 8 schematically shows an enlarged jaw portion of a fixation tool according to an embodiment with an indication of a width 80 of the opening of the jaw portion.

In the above example of the fixation tool adapted for use in connection with IS1 and DS4 terminal pins, the width 80 (i.e., distance between the opposing inner surfaces of the first and second jaws may range between 1mm and 0.8mm e.g. to obtain the desired click function.

Fig. 9 schematically shows a perspective view of a cavity system at a jaw portion of a fixation tool according to an embodiment with inclined opposing inner jaw surfaces.

As shown in Fig. 9, the opposing inner surfaces of the first and second jaws are inclined so that the distance between the surfaces linearly increases from the side edges to the middle of the surfaces. Thereby, insertion of the lead terminal pins can be facilitated.

In the above example of the fixation tool adapted for use in connection with IS1 and DS4 terminal pins, the distance M at the middle may range between 1.39mm and 1.60mm and the distance S at the side edges may respectively range between 1.61mm and 1.82mm at a handle compression force of about 15N.

Alternatively, other non-linear inclinations (quadratic, circular etc.) may be used to facilitate insertion of the terminal pins.

Fig. 10 shows an example of a fixation tool with an insertion support funnel 100 according to an embodiment related to the above-mentioned stylet (e.g., stylet 30 in Fig. 1A) inserted from the proximal end to the distal end of the lead to assist in positioning the distal end and the helix within the heart. The insertion support funnel 100 facilitates the insertion of the stylet into the respective aperture of the lead terminal pin after being clamped in the cavity system, e.g., at the first cavity.

Thereby, a physician or other user that is not yet familiar with the fixation tool and its pin insertion area can be supported by the insertion support funnel 100 to guide the distal end (e.g., mandrel) of the stylet into the aperture of the lead terminal pin clamped by the cavity system of the jaw portion. In case of the small design of the cavity system for IS1 and DF4 lead terminal pins, the area (e.g., first cavity) dedicated to clipping the IS1 or DF4 pin is barely visible to the physician or other user and may be confused e.g. with the distal stress relieving element 54 (fourth cavity). The insertion support funnel prevents visual confusion so that the physician or other user will not hesitate to insert the distal end of the stylet into the aperture of the clamped lead terminal pin by a forced axial movement, since the risk of breaking the terminal pin and/or an inner pin connection is reduced.

The insertion support funnel 100 may be provided as single-part element molded or removably fixed (e.g., clipped) either to the first jaw or the second jaw, or as a twopart element with a first part molded or removably fixed to the first jaw and a second part molded or removably fixed to the second jaw. The insertion support funnel 100 may be fixed to the fixation tool either during manufacturing/assembly of the fixation tool or during use by the physician or other user for increased flexibility of use (e.g., optional use, pin-dependent use etc.).

In an example, a "dual-use" insertion support funnel 100 may be provided, that is capable of being used in a first mode (inserted directly on the lead terminal pin) or in a second mode ("plugged" on the fixation tool).

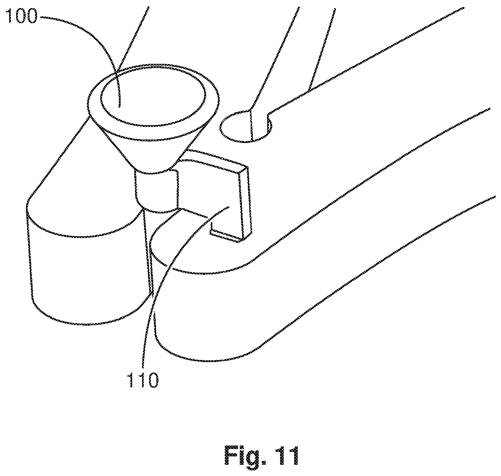

Fig. 11 schematically shows a perspective view and a front view of a fixation tool with an alternative design of the insertion support funnel 100.

The insertion support funnel 100 shown in Fig. 11 consists of two separated molded plastic parts, the funnel 100 and a holding element 102, which may be permanently assembled during manufacturing (e.g., by molding) or plugged together on the sterile field by a physician or other user. This option allows a non-interrupted 360° profile of the insertion support funnel 100. Moreover, the geometrical design allows for alignment of the axis of the insertion support funnel 100 with the clamp axis of the jaw portion.

As an additional or alternative measure, a visible arrow-type mark specifying the proper clipping area (e.g., first cavity) could also provide support for proper insertion of the stylet.

The proposed designs of the insertion support funnel 100 of Figs. 10 and 11 is very flexible in its use and does not lead to any ergonomic drawbacks compared to the other embodiments discussed herein. In addition to facilitating the insertion of the lead terminal pins, it clearly indicates to the user the clipping area where the stylet is to be inserted.

The asymmetry introduced by the insertion support funnels 100 of Figs. 10 and 11 may introduce a risk of use, manufacturing and/or assembling errors. In alternative embodiments, the insertion support funnel 100 may therefore be provided on both sides to obtain a symmetrical overall structure.

It is noted that the above insertion support funnels 100 of Figs. 10 and 11 may as well be provided for fixation tools with a jaw portion adapted for clamping a single type of lead terminal pin, i.e., a jaw portion with a single cavity shaped for accommodating and clamping terminal pins of a single diameter.

To summarize, a fixation tool for clamping and rotating a lead terminal pin of a medical implantable cardiac lead has been described, which comprises a handle portion and a jaw portion, wherein opposing inner surfaces of a pair of jaws of the jaw portion are shaped to form a cavity system between the pair of jaws, the cavity system comprising a first cavity located proximal to an opening at the distal end of the jaw portion and having a first width between the opposing inner surfaces and being shaped to clamp a first lead terminal pin of a first diameter, and a second cavity located proximal to the first cavity and having a second width between the opposing inner surfaces, smaller than the first width and adapted to clamp, together with the first cavity, a second lead terminal pin of a second diameter larger than the first diameter.

The embodiments and examples described herein are to be understood as illustrative examples of embodiments of the invention. Further embodiments and examples are envisaged. As already mentioned, the shape and number of cavities for different terminal pins with different diameters and/or shapes may vary allow clamping the more or other terminal pins by using the same single fixation tool.

Any feature described in relation to any one example or embodiment may be used alone or in combination with other features. In addition, any feature described in relation to any one example or embodiment may also be used in combination with one or more features of any other of the examples or embodiments, or any combination of any other of the examples or embodiments. Furthermore, equivalents and modifications not described herein may also be employed within the scope of the invention as defined in the claims.

## Claims

1. A fixation tool (40) for clamping a terminal pin (70, 72) of a medical implantable cardiac lead (20), said fixation tool (40) comprising:
a handle portion comprising a pair of arms formed by a first arm (43(1)) connected at a connecting hinge portion (3) to a second arm (43(2)), and
a jaw portion comprising a pair of jaws (45(1), 45(2)) for receiving the terminal pin (70, 72) between respective inner surfaces of the pair of jaws (45(1), 45(2)),
the connecting hinge portion (3) being arranged between the jaw portion and the handle portion,
wherein a pressure exerted on the first and second arms (43(1), 43(2)) causes an opening of the pair of jaws (45(1), 45(2)) by means of a pivotal movement of the first arm (43(1)) and the second arm (43(2)) about the connecting hinge portion (3); and
wherein opposing inner surfaces of the pair of jaws (43(1), 43(2)) are shaped to form a cavity system (1) between the pair of jaws (43(1), 43(2)), the cavity system (1) comprising a first cavity (51) located proximal to an opening (2) at the distal end of the jaw portion and having a first width between the opposing inner surfaces and being shaped to clamp a first lead terminal pin (72) of a first diameter;
**characterized in that** the cavity system (1) further comprises:
a second cavity (52) located proximal to the first cavity (51) and having a second width between the opposing inner surfaces, smaller than the first width and adapted to clamp, together with the first cavity (51), a second lead terminal pin (70) of a second diameter larger than the first diameter; and
a third cavity (53) located proximal to the second cavity (52) and having a third width smaller than the first diameter.

2. The fixation tool (40) according to claim 1, wherein the cavity system (1) comprises a fourth cavity (54) located proximal to the third cavity (53) and forming a proximal end of the cavity system (1) and having a width larger than the third width.

3. The fixation tool (40) according to any one of the preceding claims, wherein each of the first and second arms (43(1), 43(2)) comprises a finger pad (42) having a concave, a convex or a flat surface at an external surface of the handle portion.

4. The fixation tool (40) according to claim 3, wherein a surface of each finger pad (42) is provided with an anti-slip surface, in particular with a ribbed surface and/or a rubber layer.

5. The fixation tool (40) according to any one of the preceding claims, wherein each of the first and second arms (43(1), 43(2)) is curved and the finger pad (42) of each of the first and second arms (43(1), 43(2)) is positioned at an inflection point of the respective arm at the handle portion.

6. The fixation tool (40) according to claim 5, wherein at the handle portion, the internal surface of each of the first and second arms (43(1), 43(2)) opposite to the finger pad (42) is provided with a concave portion adapted to receive a finger (12) for rotating the fixation tool (40) with respect to the terminal pin (70, 72).

7. The fixation tool (40) according to one of the preceding claims, wherein the internal surface of the first arm (43(1)) is provided with a first protrusion (44; 46) and the internal surface of the second arm (43(2)) is provided with a second protrusion (44; 46), the first protrusion (44; 46) and the second protrusion (44; 46) being arranged such that a predetermined pressure on the handle portion causes said protrusions (44; 46) to abut against each other to provide a stopping function.

8. The fixation tool (40) according to claim 7, wherein the first protrusion (44) and the second protrusion (44) are arranged at a free end of the handle portion.

9. The fixation tool (40) according to claim 7, wherein the first protrusion (46) and the second protrusion (46) are arranged between the finger pads (42) and the connecting hinge portion (3).

10. The fixation tool (40) according to any one of the preceding claims, wherein the opening of the distal end (2) of the jaw portion when no pressure is applied to the handle portion is smaller than the second diameter.

11. The fixation tool (40) according to any one of the preceding claims, wherein the first diameter ranges between 1.65mm and 1.50mm, in particular between 1.62mm and 1.56mm, and the second diameter ranges between 1.50mm and 1.30mm, in particular between 1.40mm and 1.34mm, when no pressure is applied to the handle portion.

12. The fixation tool (40) according to claim 12, wherein the minimum distance between the opposing inner surfaces of the pair of jaws (45(1), 45(2)) at the opening (2) of the distal end ranges between 0.50mm and 1.20mm, in particular between 0.80mm and 1.00mm, when no pressure is applied to the handle portion.

13. The fixation tool (40) according to any one of the preceding claims, being integrally made in one-piece, in particular of a plastic material.

14. The fixation tool (40) according to any one of the preceding claims, further comprising an insertion support funnel (100) removably or non-removably fixed to the jaw portion to facilitate insertion of a stylet into the cardiac lead (20) via an aperture of the lead terminal pin (70, 72).

## Patentansprüche

1. Fixierungswerkzeug (40) zum Klemmen eines Anschlussstiftes (70, 72) einer medizinischen implantierbaren Herzsonde (20), wobei das besagte Fixierungswerkzeug (40) umfasst:
einen Griffabschnitt, der ein Armpaar umfasst, das durch einen ersten Arm (43(1)) ausgebildet ist, der an einem Verbindungsscharnierabschnitt (3) mit einem zweiten Arm (43(2)) verbunden ist, und
einen Backenabschnitt, der ein Paar Backen (45(1), 45(2)) zur Aufnahme des Anschlussstiftes (70, 72) zwischen den jeweiligen Innenflächen des Paares von Backen (45(1), 45(2)) umfasst,
wobei der Verbindungsscharnierabschnitt (3) zwischen dem Backenabschnitt und dem Griffabschnitt angeordnet ist,
wobei ein auf den ersten und zweiten Arm (43(1), 43(2)) ausgeübter Druck ein Öffnen des Paares von Backen (45(1), 45(2)) durch eine Schwenkbewegung des ersten Arms (43(1)) und des zweiten Arms (43(2)) um den Verbindungsscharnierabschnitt (3) bewirkt; und
wobei gegenüberliegende Innenflächen des Paares von Backen (43(1), 43(2)) so ausgebildet sind, dass sie ein Hohlraumsystem (1) zwischen dem Paar von Backen (43(1), 43(2)) bilden, wobei das Hohlraumsystem (1) einen ersten Hohlraum (51) umfasst, der proximal zu einer Öffnung (2) am distalen Ende des Backenabschnitts angeordnet ist und eine erste Breite zwischen den gegenüberliegenden Innenflächen aufweist und so geformt ist, dass er einen ersten Leitungsanschlussstift (72) mit einem ersten Durchmesser einklemmt;
**dadurch gekennzeichnet, dass** das Hohlraumsystem (1) ferner umfasst:
einen zweiten Hohlraum (52), der proximal zum ersten Hohlraum (51) angeordnet ist und eine zweite Breite zwischen den gegenüberliegenden Innenflächen aufweist, die kleiner als die erste Breite ist und angepasst ist, um zusammen mit dem ersten Hohlraum (51) einen zweiten Leitungsanschlussstift (70) mit einem zweiten Durchmesser, der größer als der erste Durchmesser ist, einzuklemmen; und
einen dritten Hohlraum (53), der proximal zum zweiten Hohlraums (52) angeordnet ist und eine dritte Breite aufweist, die kleiner als der erste Durchmesser ist.

2. Fixierungswerkzeug (40) nach Anspruch 1, wobei das Hohlraumsystem (1) einen vierten Hohlraum (54) umfasst, der proximal zum dritten Hohlraum (53) angeordnet ist und ein proximales Ende des Hohlraumsystems (1) bildet und eine größere Breite als die dritte Breite aufweist.

3. Fixierungswerkzeug (40) nach einem der vorhergehenden Ansprüche, wobei jeder der ersten und zweiten Arme (43(1), 43(2)) ein Fingerpad (42) mit einer konkaven, einer konvexen oder einer flachen Fläche an einer Außenfläche des Griffabschnitts umfasst.

4. Fixierungswerkzeug (40) nach Anspruch 3, wobei eine Fläche jedes Fingerpads (42) mit einer rutschhemmenden Fläche, insbesondere mit einer gerippten Fläche und/oder einer Gummischicht, versehen ist.

5. Fixierungswerkzeug (40) nach einem der vorhergehenden Ansprüche, wobei jeder der ersten und zweiten Arme (43(1), 43(2)) gekrümmt ist und das Fingerpad (42) jedes der ersten und zweiten Arme (43(1), 43(2)) an einem Wendepunkt des jeweiligen Arms am Griffabschnitt positioniert ist.

6. Fixierungswerkzeug (40) nach Anspruch 5, wobei am Griffabschnitt die Innenfläche jedes der ersten und zweiten Arme (43(1), 43(2)) gegenüber des Fingerpads (42) mit einem konkaven Abschnitt versehen ist, der angepasst ist, um einen Finger (12) zum Drehen des Fixierungswerkzeugs (40) in Bezug auf den Anschlussstift (70, 72) aufzunehmen.

7. Fixierungswerkzeug (40) nach einem der vorhergehenden Ansprüche, wobei die Innenfläche des ersten Arms (43(1)) mit einem ersten Vorsprung (44; 46) und die Innenfläche des zweiten Arms (43(2)) mit einem zweiten Vorsprung (44; 46) versehen ist, wobei der erste Vorsprung (44; 46) und der zweite Vorsprung (44; 46) so angeordnet sind, dass ein vorbestimmter Druck auf den Griffabschnitt bewirkt, dass die besagten Vorsprünge (44; 46) aneinander stoßen, um eine Stoppfunktion bereitzustellen.

8. Fixierungswerkzeug (40) nach Anspruch 7, wobei der erste Vorsprung (44) und der zweite Vorsprung (44) an einem freien Ende des Griffabschnitts angeordnet sind.

9. Fixierungswerkzeug (40) nach Anspruch 7, wobei der erste Vorsprung (46) und der zweite Vorsprung (46) zwischen den Fingerpads (42) und dem Verbindungsscharnierabschnitt (3) angeordnet sind.

10. Fixierungswerkzeug (40) nach einem der vorhergehenden Ansprüche, wobei die Öffnung des distalen Endes (2) des Backenabschnitts, wenn kein Druck auf den Griffabschnitt ausgeübt wird, kleiner ist als der zweite Durchmesser.

11. Fixierungswerkzeug (40) nach einem der vorhergehenden Ansprüche, wobei der erste Durchmesser zwischen 1,65 mm und 1,50 mm, insbesondere zwischen 1,62 mm und 1,56 mm, und der zweite Durchmesser zwischen 1,50 mm und 1,30 mm, insbesondere zwischen 1,40 mm und 1,34 mm, liegt, wenn kein Druck auf den Griffabschnitt ausgeübt wird.

12. Fixierungswerkzeug (40) nach Anspruch 12, wobei der Mindestabstand zwischen den gegenüberliegenden Innenflächen des Backenpaares (45(1), 45(2)) an der Öffnung (2) des distalen Endes zwischen 0,50 mm und 1,20 mm, insbesondere zwischen 0,80 mm und 1,00 mm liegt, wenn kein Druck auf den Griffabschnitt ausgeübt wird.

13. Fixierungswerkzeug (40) nach einem der vorhergehenden Ansprüche, das einteilig, insbesondere aus einem Kunststoffmaterial, hergestellt ist.

14. Fixierungswerkzeug (40) nach einem der vorhergehenden Ansprüche, ferner umfassend einen Einsetzstütztrichter (100), der abnehmbar oder nicht abnehmbar am Backenabschnitt befestigt ist, um das Einsetzen eines Stiletts in die Herzsonde (20) über eine Öffnung des Leitungsanschlussstiftes (70, 72) zu erleichtern.

## Revendications

1. Outil de fixation (40) permettant de serrer une broche de borne (70, 72) d'une sonde cardiaque implantable médicale (20), ledit outil de fixation (40) comprenant :
une partie de poignée comprenant une paire de bras formés par un premier bras (43(1)) relié à une partie de charnière de connexion (3) à un second bras (43(2)), et
une partie de mâchoire comprenant une paire de mâchoires (45(1), 45(2)) pour recevoir la broche de borne (70, 72) entre les surfaces intérieures respectives de la paire de mâchoires (45(1), 45(2)),
la partie de charnière de connexion (3) est disposée entre la partie de mâchoire et la partie de poignée,
dans lequel une pression exercée sur le premier et le second bras (43(1), 43(2)) provoque l'ouverture de la paire de mâchoires (45(1), 45(2)) au moyen d'un mouvement de pivotement du premier bras (43(1)) et du second bras (43(2)) autour de la partie de charnière de connexion (3) ; et
dans lequel les surfaces intérieures opposées de la paire de mâchoires (43(1), 43(2)) sont façonnées pour former un système de cavité (1) entre la paire de mâchoires (43(1), 43(2)), le système de cavité (1) comprenant une première cavité (51) située à proximité d'une ouverture (2) à l'extrémité distale de la partie de mâchoire et ayant une première largeur entre les surfaces intérieures opposées et étant façonnée pour serrer une première broche de borne de connexion (72) d'un premier diamètre ;
**caractérisé en ce que** le système de cavité (1) comprend en outre :
une seconde cavité (52) située à proximité de la première cavité (51) et présentant une seconde largeur entre les surfaces intérieures opposées, inférieure à la première largeur et adaptée pour serrer, avec la première cavité (51), une seconde broche de borne de connexion (70) d'un second diamètre supérieur au premier diamètre ; et
une troisième cavité (53) située à proximité de la seconde cavité (52) et présentant une troisième largeur inférieure au premier diamètre.

2. Outil de fixation (40) de la revendication 1, dans lequel le système de cavité (1) comprend une quatrième cavité (54) située à proximité de la troisième cavité (53) et formant une extrémité proximale du système de cavité (1) et présentant une largeur supérieure à la troisième largeur.

3. Outil de fixation (40) de l'une quelconque des revendications précédentes, dans lequel chacun des premier et second bras (43(1), 43(2)) comprend un bout de doigt (42) présentant une surface concave, convexe ou plate au niveau d'une surface externe de la partie de poignée.

4. Outil de fixation (40) de la revendication 3, dans lequel une surface de chaque bout de doigt (42) est pourvue d'une surface antidérapante, notamment d'une surface nervurée et/ou d'une couche de caoutchouc.

5. Outil de fixation (40) de l'une quelconque des revendications précédentes, dans lequel chacun des premier et second bras (43(1), 43(2)) est incurvé et le bout de doigt (42) de chacun des premier et second bras (43(1), 43(2)) est positionné à un point d'inflexion du bras respectif au niveau de la partie de poignée.

6. Outil de fixation (40) de la revendication 5, dans lequel au niveau de la partie de poignée, la surface interne de chacun des premier et second bras (43(1), 43(2)) opposés au bout de doigt (42) est pourvue d'une partie concave adaptée pour recevoir un doigt (12) pour faire tourner l'outil de fixation (40) par rapport à la broche de borne (70, 72).

7. Outil de fixation (40) de l'une des revendications précédentes, dans lequel la surface interne du premier bras (43(1)) est pourvue d'une première saillie (44 ; 46) et la surface interne du second bras (43(2)) est pourvue d'une seconde saillie (44 ; 46), la première saillie (44 ; 46) et la seconde saillie (44 ; 46) étant disposées de telle sorte qu'une pression prédéterminée sur la partie de poignée amène lesdites saillies (44 ; 46) à venir en butée l'une contre l'autre afin d'assurer une fonction d'arrêt.

8. Outil de fixation (40) de la revendication 7, dans lequel la première saillie (44) et la seconde saillie (44) sont disposées à une extrémité libre de la partie de poignée.

9. Outil de fixation (40) de la revendication 7, dans lequel la première saillie (46) et la seconde saillie (46) sont disposées entre les bouts de doigts (42) et la partie de charnière de connexion (3).

10. Outil de fixation (40) de l'une quelconque des revendications précédentes, dans lequel l'ouverture de l'extrémité distale (2) de la partie de mâchoires lorsqu'aucune pression n'est appliquée sur la partie de poignée est inférieure au second diamètre.

11. Outil de fixation (40) de l'une quelconque des revendications précédentes, dans lequel le premier diamètre est compris entre 1,65 mm et 1,50 mm, notamment entre 1,62 mm et 1,56 mm, et le second diamètre est compris entre 1,50 mm et 1,30 mm, notamment entre 1,40 mm et 1,34 mm, lorsqu'aucune pression n'est appliquée sur la partie de poignée.

12. Outil de fixation (40) de la revendication 12, dans lequel la distance minimale entre les surfaces intérieures opposées de la paire de mâchoires (45(1), 45(2)) au niveau de l'ouverture (2) de l'extrémité distale est comprise entre 0,50 mm et 1,20 mm, notamment entre 0,80 mm et 1,00 mm, lorsqu'aucune pression n'est appliquée sur la partie de poignée.

13. Outil de fixation (40) de l'une quelconque des revendications précédentes, étant réalisé d'une seule pièce, notamment en matière plastique.

14. Outil de fixation (40) de l'une quelconque des revendications précédentes, comprenant en outre un entonnoir de support d'insertion (100) fixé de manière amovible ou non à la partie de mâchoires pour faciliter l'insertion d'un stylet dans la sonde cardiaque (20) via une ouverture de la broche de borne de connexion (70, 72).
